Europäisches Patentamt

European Patent Office

Office européen des brevets

(19)

(11) Veröffentlichungsnummer: **0 078 930**
**B1**

(12) **EUROPÄISCHE PATENTSCHRIFT**

(45) Veröffentlichungstag der Patentschrift:
19.03.86

(51) Int. Cl.⁴: **A 61 F 5/44**

(21) Anmeldenummer: **82109407.5**

(22) Anmeldetag: **12.10.82**

(54) **Medizinischer Auffangbeutel.**

(30) Priorität: **20.10.81 DE 3141566**

(43) Veröffentlichungstag der Anmeldung:
**18.05.83 Patentblatt 83/20**

(45) Bekanntmachung des Hinweises auf die Patenterteilung:
**19.03.86 Patentblatt 86/12**

(84) Benannte Vertragsstaaten:
**DE FR IT SE**

(56) Entgegenhaltungen:
**EP - A - 0 013 109**
**DE - A - 2 829 332**
**GB - A - 1 370 622**
**US - A - 3 421 506**
**US - A - 3 690 320**
**US - A - 3 825 005**

(73) Patentinhaber: **Beiersdorf Aktiengesellschaft,
Unnastrasse 48, D-2000 Hamburg 20 (DE)**

(72) Erfinder: **Ganz, Franz-Josef, Dr.med., Nobelstrasse 7,
D-5090 Leverkusen (DE)**
Erfinder: **Mundt, Michael, Dipl.-Kaufmann,
Joachim-Mähl-Strasse 26, D-2000 Hamburg 61 (DE)**

LIBER, STOCKHOLM 1986

## Beschreibung

Die Erfindung bezieht sich auf einen medizinischen Auffangbeutel, d.h. Ileo-, Colo-, Urostomiebeutel oder dgl., mit einer durch eine Einlaßöffnung zugänglichen, im wesentlichen sonst dichten Beutelkammer zur Aufnahme der jeweiligen Ausscheidungsprodukte, welche eine von außerhalb der Beutelkanner zu öffnende Verschlußkonstruk-tion für eine Auslaßöffnung aufweist, sowie zweckmäßig nit einer Halte- bzw. Befestigungsvorrichtung für den Beutel.

Besonders bei Patienten mit künstlichem Darmausgang werden solche Beutel mit Hilfe der Befestigungsvorrichtung in Form eines Haftringes an der Bauchdecke befestigt. Es waren bisher einerseits Beutel vorgeschlagen worden, die eine wiederverschließbare Auslaßöffnung aufweisen, z.B. nach US Patent 3,825,005, mit einem an sich bekannten Mikro-Snap-Verschluß. Ein solcher Beutel ist jedoch relativ aufwendig teuer und deshalb nicht als preiswerter Wegwerfartikel geeignet. Außerdem ist der Verschluß nicht gegen unbeabsichtigtes Offnen geschützt. Da zudem bei jedem Entleerungsvorgang die Auslaßöffnung und damit die Rillen des Verschlusses verunreinigt werden, leidet nicht nur die Dichtigkeit beim Wiederverschließen, sondern es kann auch eine Beschmutzung der Finger beim Wiederöffnen vor dem nächsten Entleerungsvorgang nicht völlig ausgeschlossen werden. Um das zu vermeiden, sind andererseits Einmal-Beutel entwickelt worden, die bisher in gefülltem Zustand über die Kanalisation wieder beseitigt wurden, was jedoch zu Verstopfungen und/oder zu einer besonderen Belastung der Kläranlagcn führte. Man ist deshalb dazu übergegangen, die Deutel vor der Beseitigung aufzuschneiden und den Inhalt herauszudrücken. Dies führt wiederum zu Beschmutzungen und stellt ebenfalls keine zufriedenstellende Lösung dar. Das gleiche Problem kann außer bei einem künstlichen Darmausgang auch bei anderen Ausscheidungsprodukten auftreten, beispielsweise nach Nieren- oder Harnblasenoperationen oder beim Auffangen der von Fisteln abfließenden Flüssigkeit.

Der Erfindung liegt die Aufgabe zugrunde, einen preiswerten Einmalartikel zu entwickeln, der die Vorteile der beiden Vorschläge miteinander verbindet, ihre Nachteile aber vermeidet, d.h., daß der Beutel sowohl vollkonmen und sicher dicht als auch einfach und ohne Beschmutzungen zu öffnen und zu entleeren sein soll. Vor allem soll dabei gleichzeitig gesichert werden, daß der Beutel nicht unbeabsichtigt geöffnet werden umd sich ungewollt entleeren kann. Erfindungsgemäß gelingt dies dadurch, daß an den Colostomie-Beutel oder dgl., der eine nur von.außerhalb der Beutelkammer zu öffnende Verschlußkonstruktion für eine Auslaßöffnung aufweist, die Verschlußkonstruktion durch eine zweite Kammer gegen unbeabsichtigtes Öffnen geschützt ist.

Im Rahmen der Erfindung wird die Konstruktion vereinfacht und in ihrer Handhabung sicherer, wemn beide Kammern von einer gemeinsamen Umfangsrandkonstruktion, insbesondere einer gemeinsamen Schweißnaht, umschlossen sind, wobei hier der Begriff "Kammer" im weitesten Sinne auch etwa als der von einer mit Öffnungen versehenen Schutzhülle eingeschlossene Raum zu verstehen ist. An sich könnte die zweite Kammer auch als Anhang an die erste Kammer ausgebildet und nur über einen schmalen Verbindungssteg mit ihr verbunden sein, der die Verschlußkonstruktion enthält. Dadurch würde aber eine ungewollte Abtrennung der zweiten Kammer begünstigt bzw. die Handhabung der Beutelkonstruktion erschwert. Überdies würde dadurch die Größe der Verschlußkonstruktion auf die Breite des schmalen Verbindungssteges reduziert. Durch die gemeinsame Schweißnaht oder sonstige Randkonstruktion wird dagegen ein handlicher und leicht herstellbarer Beutel mit platz auch für einen breiten Verschluß geschaffen, durch den auch breiige Substanzen leicht hindurchgedrückt werden können.

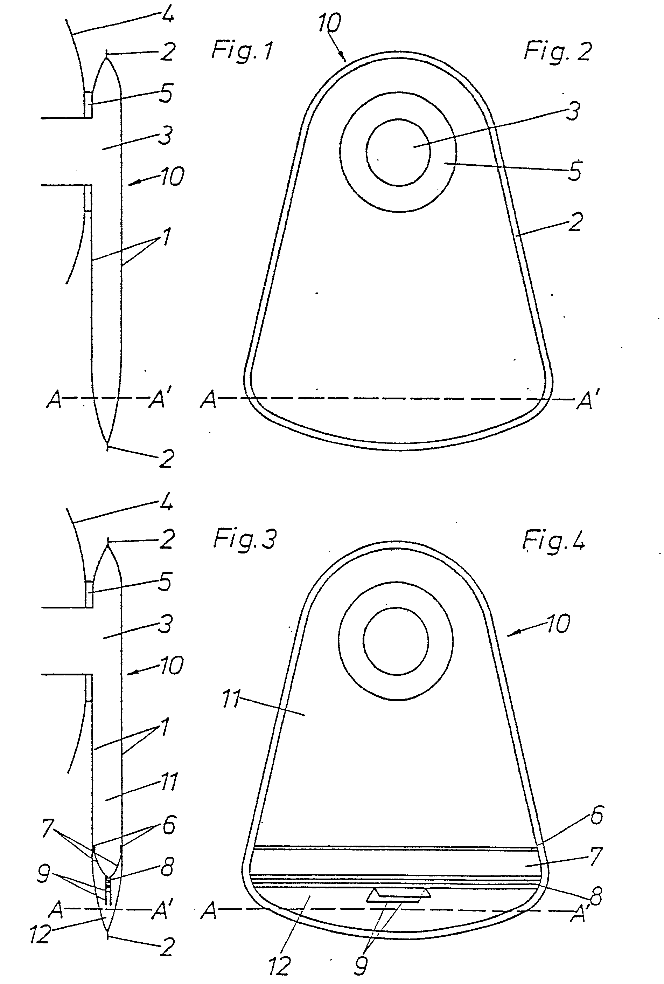

Weitere Einzelheiten der Erfindung ergeben sich an Hand der nachfolgenden Beschreibung des Standes der Technik (Figuren 1 und 2), einer Ausführungsform der Erfindung (Figuren 3 und 4), die jeweils in einem schematischen Schnitt und in einem Aufriß dargestellt sind und einer weiteren Ausführungsform der Erfindung (Figur 5).

Ein bekannter Colostomie-Beutel 10 besteht aus zwei Folienzuschnitten 1, die über eine Umfangs-schweißnaht 2 miteinander verbunden sind. In einer der Folien 1 ist eine Öffnung 3 vorgesehen, um deren Rand ein auf der Haut der Bauchdecke 4 eines Patienten haftender Ring 5 angeordnet ist. Für andere Typen von Beutelkonstruktionen sind auch andere Halte- bzw. Befestigungsvorrichtungen bekannt geworden, z.B. Ösen zum Anhängen des Beutels.

Nach dem Füllen des Beutels 10 wird dieser entfernt und soll vor seiner Beseitigung beispielsweise entlang der Linie A - A' aufgeschnitten und sein Inhalt ausgedrückt werden. Es ist verständlich, daß es dabei leicht zu Beschmutzungen kommt, insbesondere aber das Schneidwerkzeug nicht sauber gehalten werden kann. Bei Beuteln zur Aufnahme von Flüssigkeiten, wie von Urin, würde der Einschnitt an der Oberseite des Beutels anstatt entlang der Linie A - A' vorgenommen werden und könnte entsprechend kleiner sein, doch wäre auch hier das Schneidwerkzeug einer gewissen Verschmutzungsgefahr ausgesetzt.

Erfindungsgemäß wird deshalb der Beutel in zwei Kammern 11 und 12 unterteilt, die voneinander durch eine Verschlußkonstruktion 8 getrennt sind, wobei die Kammer 11 mit der Einlaßöffnung 3 versehen ist. Die Kammer 12 dient dabei als Hüllraum zum Schutze der Verschlußkonstruktion 8, um den Zutritt zu dieser und damit ein unbeabsichtigtes Öffnen derselben zu verhindern.

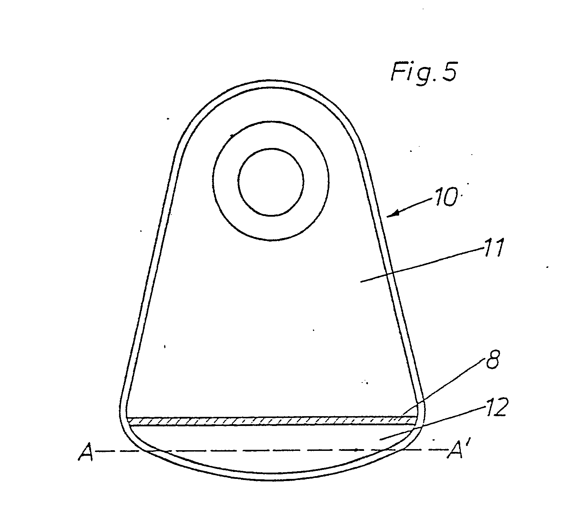

Die Verschlußkonstruktion 8 kann an sich beliebiger Art sein und beispielsweise aus einer mittels eines Stöpsels, insbesondere eines Schraubstöpsels ver-schließbaren Öffnung oder einem Aufreißverschluß bestehen. Eine mittels Stöpsel verschließbare Öffnung erfordert jedoch einen verstärkten Öffnungsrand, der die Beutelkonstruktion verdickt und deshalb beim Lagern etwas platzaufwendig sein kann. Eine platzsparende und einfache Konstruktion ist dagegen in den Figuren 3 und 4 dargestellt, bei welcher in den Beutel 10 zwei Folienstreifen 7 entlang je einer Schweißnaht 6 eingeschweißt sind. Die beiden Folienstreifen 7 weisen an ihren einander zugekehrten Seiten die beiden Teile (Feder und Nut) eines an sich bekannten, nur von einer Seite öffenbaren Gleitverschlusses 8 auf, der sich zweckmäßig über die gesamte Länge in einer Dimension des Beutels 10 erstreckt, gemäß der Darstellung der Figur 4 also im wesentlichen über die gesamte Beutelbreite, um so nach dem Auseinanderziehen eine große Öffnung zu bilden und ein rasches Entfernen des Beutelinhalts zu ermöglichen.

Zum leichteren Öffnen des Gleitverschlusses 8 sind zwei Betätigungslaschen 9 vorgesehen, die in die Kammer 12 ragen und daher normalerweise nicht zugänglich sind. Sobald die Kammer 11 des Beutels 10 gefüllt und der Beutel 10 entfernt werden soll, braucht nur die leer gebliebene Kammer 12 entlang der Linie A - A' aufgeschnitten werden, was ohne Gefahr einer Verschmutzung geschehen kann. Nach dem Öffnen des Verschlusses 8 kann der Beutelinhalt leicht gedrückt werden, worauf der Beutel - gegebenenfalls nach neuerlichem Verschließen des Verschlusses 8 durch leichtes Darüberstreichen von der sauberen Außen-seite her - zum Abfall gegeben werden kann.

In einer weiteren, vereinfachten Ausführungsform der Erfindung (dargestellt in Figur 5) ist die Ver-schlußkonstruktion 8 durch einen beidseitig selbstklebenden schmalen Klebestreifen (etwa 0,5 - 1 cm breit) gebildet, der vorzugsweise über die gesamte Breite des Beutels angebracht ist, d.h. von Schweißnaht zu Schweißnaht verläuft. Er verschließt auf diese Weise die Beutelkammer 11 an ihrem unteren Ende und bildet gleichzeitig die Trennlinie zwischen den beiden Kammern 11 und 12. Zum Öffnen und Entleeren des Beutels wird wiederum nur die Kammer 12 entlang der Linie A - A' aufgeschnitten und durch Zug an den verbliebenen Seitenwandresten dieser Kammer die durch den Klebestreifen gebildete Klebnaht aufgerissen.

Zur völligen Beseitigung des Auffangbeutels nach der Benutzung empfiehlt es sich, die Wandungen zumindest eines Teiles des Beutels 10 aus verrottbarem Kunststoffmaterial herzustellen, soweit die Forderungen nach Dichtigkeit und Raschelarmut noch erfüllt sind. Im vorliegenden Anwendungsfalle wird nämlich die Verrottung durch die Reste des in die Kammer 11 eingebrachten Inhalts beschleunigt.

Aus der obigen Beschreibung ist verständlich, daß die Verschlußkonstruktion nicht nur verschieden ausgebildet, sondern auch an verschiedenen Stellen des Beutels 10, immer jedoch geschützt durch die Kammer 12, angeordnet sein kann. Es ist vorteilhaft, wenn die Kammern 11 und 12 entsprechend Figur 4 von einer gemeinsamen, im wesentlichen krümmungsfrei verlaufenden Schweißnaht 2 umrandet sind und nicht zwischen beiden Kammern ein Einschnitt vorgesehen ist, der die Breite des Verschlusses 8 nur begrenzen und die Handhabung erschweren würde. Es kann aber beispielsweise entlang der Linie A - A' eine Solltrennstelle in Form einer Perforation oder einer sonstwie in bekannter Weise geschwächten Stelle (dünneres Material oder Material geringerer Festigkeit) vorgesehen sein. Dabei ist der Perforation insofern der Vorzug zu geben, weil das Anbringen von kleinen Öffnungen in der Kammer 12 für das Verpacken nach der Herstellung der Beutel 10 zweckmäßig sein kann. Durch solche Öffnungen kann nämlich nach dem Verschließen des Verschlusses 8 oder eines anderen Verschlusses etwaige eingeschlossene Luft entweichen, die andernfalls ein flaches Lagern der Beutel verhindern würde.

### Patentansprüche

1. Medizinischer Einmal-Auffangbeutel (10) mit einer durch eine Einlaßöffnung (3) zugänglichen, im wesentlichen dichten Beutelkammer (11) zur Aufnahme von Ausscheidungsprodukten sowie zweckmäßig mit einer Haltebzw. Befestigungsvorrichtung (5) für den Beutel, welcher Beutel eine nur von außerhalb der Beutelkammer zu öffnende Verschlußkonstruktion (8) für eine Auslaß-öffnung aufweist, dadurch gekennzeichnet, daß die Verschlußkonstruktion (8) durch eine zweite Kammer (12) gegen unbeabsichtigtes Öffnen geschützt ist.

2) Auffangbeutel gemäß Anspruch 1, dadurch gekennzeichnet, daß beide Kammern (11 und 12) von einer gemeinsamen Umfangsrandkonstruktion, insbesondere von einer gemeinsamen Schweißnaht (2), umschlossen sind.

3) Auffangbeutel gemäß Anspruch 1 oder 2, dadurch gekennzeichnet, daß die Verschlußkonstruktion mit Hilfe einer Betätigungseinrichtung (9) zu öffnen ist, die sich innerhalb der zweiten Kammer (12) befindet.

4) Auffangbeutel gemäß Anspruch 1 oder 2, dadurch gekennzeichnet, daß die Verschlußkonstruktion (8) durch einen beidseitig selbstklebenden Klebestreifen gebildet ist.

5) Auffangbeutel gemäß Anspruch 1, 2, 3 oder 4, dadurch gekennzeichnet, daß für das Öffnen der zweiten Kammer (12) eine geschwächte Solltrennstelle (vgl. A - A') vorgesehen ist.

## Claims

1. Medical disposable collection bag (10) with a substantially impervious bag chamber (11), accessible through an inlet opening (3), for receiving excreta and advantageously with a holding or fixing device (5) for the bag, which bag has a closure construction (8), which can be opened only from the outside of the bag chamber,for an outlet opening, characterised in that the closure construction (8) is protected by a second chamber (12) from inadvertent opening.

2. Collection bag according to Claim 1, characterised in that the two chambers (11 and 12) are surrounded by a common peripheral edge construction, in particular a common weld seam (2).

3. Collection bag according to Claim 1 or 2, characterised in that the closure construction can be opened by means of an actuating device (9) which is located inside the second chamber (12).

4. Collection bag according to Claim 1 or 2, characterised in that the closure construction (8) is formed by an adhesive tape which is self-adhesive on both sides.

5. Collection bag according to Claim 1, 2, 3 or 4, characterised in that a weakened predetermined break point (cf. A-A') for opening the second chamber (12) is provided.

## Revendications

1. Sac collecteur médical (10) à jeter, avec une poche de sac (11) essentiellement étanche accessible par un orifice d'entrée (3), pour recueillir les excréments, ainsi qu'avantageusement avec un dispositif de maintien ou de fixation (5) du sac, qui présente un dispositif d'obturation (8) d'un orifice d'évacuation, s'ouvrant uniquement de l'extérieur de la poche de sac, caractérisé en ce que le dispositif d'obturation (8) est protégé par une deuxième poche (12) contre une ouverture imprévue.

2. Sac collecteur suivant la revendication 1, caractérisé en ce que les deux poches (11 et 12) sont entourées par une structure périphérique commune, en particulier par un cordon de soudure (2) commun.

3. Sac collecteur suivant la revendication 1 ou 2, caractérisé en ce que le dispositif d'obturation peut s'ouvrir à l'aide d'un système de commande (9) qui se trouve à l'intérieur de la deuxième poche (12).

4. Sac collecteur suivant la revendication 1 ou 2, caractérisé en ce que le dispositif d'obturation (8) est formé par une bande auto-adhésive sur ses deux faces.

5. Sac collecteur suivant la revendication 1, 2, 3 ou 4, caractérisé en ce qu'il est prévu une zone de séparation affaiblie (voir A-A') pour l'ouverture de la deuxième poche (12).

Fig.1   Fig. 2   Fig.3   Fig.4

-2/2

Fig.5

10

11

8

12

A — — — — — — — — — — — — — — — — — — — — — — A'